# EUROPEAN PATENT APPLICATION

(11) **EP 3 721 717 A1**
(43) Date of publication of application: **14.10.2020**
(21) Application number: 19208311.1
(22) Date of filing: 11.11.2019
(51) Int. Cl.: A23K 10/12, A23K 10/24, A23K 50/75

(54) **METHOD OF MANUFACTURING FERMENTED PORCINE BLOOD AND ANTIBACTERIAL COMPOSITION AND POULTRY FEED COMPOSITION USING SAME**

(30) Priority: 12.04.2019 KR 20190043318; 12.04.2019 KR 20190043319
(71) Applicant: Samda Co., Ltd., Jejudo 63016 (KR)
(72) Inventor: YU, Haeng Soo, 63035 Jeju-si, Jeju-do (KR); MOON, Sang Wook, Jeju-si, Jeju-do 63174 (KR); KIM, Chin Kyung, 63337 Jeju-si, Jeju-do (KR); PARK, Eum Mi, 05507 Seoul (KR); KIM, Won Ju, 46533 Busan-si (KR); SHIN, Eun Chule, 50658 Kyungsangnam-do (KR); KIM, Yeong Woo, 49092 Busan-si (KR); KIM, Jae Hwang, Jeju-si, Jeju-do 63047 (KR); YANG, Bong Kyu, 63243 Jeju-si, Jeju-do (KR)
(74) Representative: Betten & Resch

(57) **Abstract**

Disclosed are a method of manufacturing fermented porcine blood having antibacterial activity and poultry egg-laying enhancement and weight gain effects, and an antibacterial composition and a poultry feed composition using the fermented porcine blood.

## Description

### BACKGROUND OF THE INVENTION

### 1. Technical Field

The present invention relates to a method of manufacturing fermented porcine blood and to an antibacterial composition and a poultry feed composition using the fermented porcine blood.

### 2. Description of the Related Art

Due to the improvement of national life and increased income, the number of slaughtered domestic cows and pigs is increasing with an increase in meat consumption. Until the early 2000s, blood byproducts were so popular that clotted blood from slaughtered cows and pigs was auctioned for sale. However, about half of available blood is not recycled, but is disposed of, due to changes in food culture.

Blood is composed primarily of proteinaceous organic materials, and rapidly decays and causes odors. In general, 5 tons of water is required to process 1 ton of animal blood, and as of 2015, the processing cost per ton is 410,000 won, and thus a processing cost of about 25 billion won is reported annually in Korea.

Therefore, if slaughterhouse blood is collected, sorted and recycled hygienically, wastewater treatment costs may be lowered and environmental pollution may be reduced. Particularly, because ocean dumping of slaughterhouse blood was banned by the London Convention, which entered into force in 2013, recycling technology is more urgently required.

As disclosed in Korean Patent No. 1012648760000 entitled "Method of manufacturing liquid fertilizer using animal blood" and Korean Patent No. 1011003290000 entitled "Amino acid using livestock blood and method of manufacturing organic fertilizer using same", research has been recently conducted to recycle slaughterhouse blood as feed or fertilizer.

The present invention discloses a technique for recycling fermented porcine blood as an antibacterial agent and poultry feed.

### SUMMARY OF THE INVENTION

Accordingly, an objective of the present invention is to provide a method of manufacturing fermented porcine blood and an antibacterial composition and a poultry feed composition using the fermented porcine blood.

Other or particular objectives of the present invention will be described below.

An aspect of the present invention pertains to a method of manufacturing fermented porcine blood.

The method of the present invention includes (a) preparing porcine blood, and (b) subjecting the porcine blood to inoculation with *Lactobacillus plantarum, Lactobacillus agilis, Lactobacillus alimentarius, Lactobacillus fermentum* or a strain mixture of *Lactobacillus fermentum* and *Saccharomyces cerevisiae,* and to fermentation culture.

In the method of the present invention, porcine blood may be blood to which a blood anticoagulant is added in order to prevent blood coagulation and to facilitate fermentation upon storage of blood because the main component thereof is protein. The blood anticoagulant may be any of those known in the art, such as sodium citrate, ACD (acid citrate dextrose), CPD (citrate phosphate dextrose), CPDA-1 (CPD adenine-1) and the like, and may be added in an amount of 2 to 15% (v/v).

In the method of the present invention, the porcine blood is preferably hydrolyzed using a proteolytic enzyme before fermentation. This hydrolysis is to prevent coagulation of the blood and to facilitate fermentation, and may be performed for a sufficient amount of time under optimal active conditions using any proteolytic enzyme. Examples of the useful proteolytic enzyme may include pepsin, trypsin, papain, bromelain, Neutrase™, Protamex™, Alcalase™, Provia™ and the like, and the optimal temperature condition for the enzyme activity is as follows: 37°C when using pepsin, 25°C when using trypsin, 45°C when using bromelain, 50°C when using Neutrase™ and Protamex™, and 60°C when using Alcalase™, and the hydrolysis time may fall in the range of 24 to 72 hr. The proteolytic enzyme may be typically added in an amount of 1 to 3% (w/v) based on the amount of the porcine blood.

In the method of the present invention, the porcine blood or the hydrolyzed porcine blood may be added with a carbon source in order to facilitate fermentation. The carbon source may be any of those known in the art, such as oligosaccharides, lactose, glucose, fructose, sugar, molasses, dextrose and the like. Taking into consideration the fermentation time, the extent of fermentation, etc., the carbon source may be added in an amount of 0.1% (w/v) to 10% (w/v) based on the amount of hydrolyzed porcine blood.

In the method of the present invention, the fermentation temperature may fall in the range of 15°C to 45°C. Taking into consideration the fermentation rate, the fermentation temperature may be set to 30°C or higher.

In the method of the present invention, the fermentation period, that is, the period of culturing of the fermentation strain after inoculation, is preferably 3 days or more. This is because the fermentation strain reaches the stable stage and the pH of 4.0 is kept constant when the fermentation period is 3 days or more, as is confirmed in the following examples. Blood samples decay rapidly if they are allowed to stand at room temperature even for only 1 - 2 days because the main component thereof is protein, but the pH of 4.0 is advantageous in that it enables long-term storage of the blood for about 12 months at room temperature.

In the method of the present invention, with reference to the following examples, it is preferred that hydrolyzed porcine blood be used, that the strain mixture be used, that 6% sugar be used as the carbon source, and that fermentation be performed at 30°C for 6 days. The fermented porcine blood thus obtained has a pH of 4.0 and may thus be stored for a long period of time, and, based on the results of elemental analysis thereof, heavy metals such as lead, mercury and the like are not detected and antibiotics are not detected at all. Accordingly, the fermented porcine blood is regarded as satisfying the Standards and Specifications of Feed and the like under the Ministry of Agriculture, Food and Rural Affairs.

Another aspect of the present invention pertains to fermented porcine blood obtained by the above method. The fermented porcine blood has antibacterial activity against E. *coli, Staphylococcus aureus, Klebsiella pneumoniae, Pseudomonas aeruginosa* and *Salmonella typhimurium* and is thus usefully added to an antibacterial composition, and moreover is useful as an additive for poultry feed because it has a poultry egg-laying stimulation effect and a poultry weight gain effect, as will be described later.

The fermented porcine blood of the present invention may be used in the form of a fermented stock solution or in the form of a liquid or powder obtained by concentrating the fermented stock solution through lyophilization, concentration under reduced pressure, vacuum drying, spray drying, hot-air drying or the like. In the case in which desired useful components of the fermented blood are concentrated and used, the fermented blood may be provided in the form of an extract. Here, the extract may be an extract obtained by extracting the fermented blood (fermented stock solution or concentrate) with water, a C1-C4 lower alcohol such as methanol, ethanol or butanol, methylene chloride, ethylene, acetone, hexane, ether, chloroform, ethyl acetate, butyl acetate, N,N-dimethylformamide (DMF), dimethyl sulfoxide (DMSO), 1,3-butylene glycol, propylene glycol or a solvent mixture thereof, an extract obtained using a supercritical extraction solvent such as carbon dioxide, pentane, etc., or a fraction obtained by fractionating the extract. The extraction process may be performed through any process such as cold extraction, refluxing, heating, sonication, supercritical extraction, etc. in consideration of the polarity of extraction material, the extent of extraction, and the extent of preservation. The extract may be used in the form of a crude extract or in the form of a liquid or powder obtained by concentrating the crude extract through lyophilization, concentration under reduced pressure, vacuum drying, spray drying, hot-air drying, or the like.

Still another aspect of the present invention pertains to an antibacterial composition containing the fermented porcine blood described above as an active ingredient.

The fermented porcine blood has antibacterial activity against *E. coli, Staphylococcus aureus, Klebsiella pneumoniae, Pseudomonas aeruginosa* and *Salmonella typhimurium,* as is confirmed in the following examples.

The antibacterial composition of the present invention may contain, based on the total weight of the composition, the fermented porcine blood as the active ingredient thereof in an amount of 0.0001 wt% to 20 wt%, and preferably 0.1 wt% to 10 wt%, depending on the type of product in which the antibacterial composition of the present invention is embodied.

In a specific embodiment, the antibacterial composition of the present invention may be a quasi-drug composition.

When the composition of the present invention is prepared as a quasi-drug composition, the composition of the present invention may be prepared so as to include a dispersant and a carrier in addition to the active ingredient.

Examples of the dispersant contained in the antibacterial composition of the present invention may include water, alcohols (e.g. methyl alcohol, ethyl alcohol, ethylene glycol, propylene glycol, diethylene glycol, glycerin, etc.), ketones (e.g. acetone, methyl ethyl ketone, etc.), ethers (e.g. dioxane, tetrahydrofuran, cellosolve, diethylene glycol dimethyl ether, etc.), aliphatic hydrocarbons (e.g. hexane, kerosene, etc.), aromatic hydrocarbons (e.g. benzene, toluene, xylene, naphthalene, methyl naphthalene, etc.), halogenated hydrocarbons (e.g. chloroform, carbon tetrachloride, etc.), acid amides (e.g., dimethyl formamide, etc.), esters (e.g. methyl acetate ester, ethyl acetate ester, butyl acetate ester, fatty acid glycerol ester, etc.), nitriles (e.g. acetonitrile, etc.), surfactants (higher alcohol sulfate esters, alkyl sulfonic acids, alkyl allyl sulfonic acids, quaternary ammonium salts, oxyalkyl amines, fatty acid esters, polyalkylene oxide compounds, and anhydro-sorbitol compounds), and the like, and these dispersants may be used alone or in combinations of two or more thereof.

Examples of the carrier contained in the antibacterial composition of the present invention may include clay (e.g. kaolin, bentonite, acid clay, etc.), talc (e.g. talc powder, feldspar powder, etc.), silica (e.g. diatomaceous earth, silicic anhydride, mica powder, etc.), alumina, sulfur powder, activated carbon, etc., and these carriers may be used alone or in combinations of two or more thereof.

When the composition of the present invention is prepared as a quasi-drug composition, it may be of any product type, so long as it complies with the enforcement regulations at the time of manufacture and distribution in legal and functional classifications. Specific examples thereof may include sanitary napkins, masks, breath fresheners, toothpaste, pads, wet tissue, sterile cotton swabs, sterile gloves and the like, manufactured through an absorption process or a coating process, or may include sterilizers/disinfectants, detergents, etc. for use in quasi-drugs for animals.

In another specific embodiment, the antibacterial composition of the present invention may be a food composition to which the antibacterial active ingredient thereof is added for preservation purposes.

The food composition of the present invention may be prepared in various forms, for example, beverages such as tea, juice, carbonated beverages, ion beverages and the like, processed milk products such as milk, yogurt and the like, foods such as gum, rice cakes, Korean traditional cookies, breads, confectionaries, noodles and the like, and functional health food formulations such as tablets, capsules, pills, granules, liquids, powders, flakes, pastes, syrups, gels, jellies, bars and the like.

Moreover, the food composition of the present invention may be of any product type, so long as it complies with the enforcement regulations at the time of manufacture and distribution in legal and functional classifications. For example, it may be a functional health food based on the Korean Health/Functional Food Act, or may be a confectionery, soy milk, tea, beverage, special-purpose food, etc. according to each food type in the Food Standards Code (published by the Ministry of Food and Drug Safety, Food Safety Standards and Specifications) under the Korea Food Sanitation Act.

The food composition of the present invention may contain a food additive in addition to the active ingredient thereof. The food additive is generally understood to be a material which is added to food and mixed or infiltrated into food in the manufacture, processing or preservation of food, and the safety thereof must be ensured because it is taken with food daily and for a long time. According to the Food Additives Code based on the laws of each country that regulate the manufacture and distribution of foods (in Korea, the Food Sanitation Act), food additives having guaranteed safety are classified in view of components or functions. Here, according to the Korea Food Additives Code (published by the Ministry of Food and Drug Safety, Food Additive Safety Standards and Specifications), food additives are classified into chemical synthetic products, natural additives, and mixed preparations depending on the components thereof. These food additives include sweeteners, flavoring agents, preservatives, emulsifiers, acidulants, and thickening agents from the viewpoint of function.

The sweetener may be used to appropriately sweeten the food, and either natural or synthetic sweeteners may be contained in the composition of the present invention. Preferably, a natural sweetener is used, examples of which include sugar sweeteners such as corn syrup solids, honey, sucrose, fructose, lactose, maltose, and the like.

The flavoring agent may be used to improve taste or flavor, and both natural and synthetic flavoring agents may be used. Preferably, a natural flavoring agent is used. When using a natural flavoring agent, the purpose of nutritional fortification may be achieved in addition to flavoring. Examples of natural flavoring agents include those obtained from apples, lemons, citrus fruits, grapes, strawberries, peaches and the like, or those obtained from green tea leaves, *Polygonatum odoratum var. pluriflorum,* bamboo leaves, cinnamon, *Chrysanthemum* leaves, jasmine and the like. Furthermore, those obtained from ginseng (red ginseng), bamboo shoots, aloe vera, ginkgo, etc. may be used. The natural flavoring agent may be a liquid concentrate or a solid extract. In some cases, a synthetic flavoring agent may be used, examples of which include esters, alcohols, aldehydes, terpenes and the like.

Examples of the preservative include sodium calcium sorbate, sodium sorbate, potassium sorbate, calcium benzoate, sodium benzoate, potassium benzoate, EDTA (ethylenediamine tetraacetic acid) and the like, and examples of the emulsifier include acacia gum, carboxymethyl cellulose, xanthan gum, pectin and the like. Examples of the acidulant include citric acid, malic acid, fumaric acid, adipic acid, gluconic acid, tartaric acid, ascorbic acid, acetic acid, phosphoric acid, and the like. The acidulant may be added such that the food composition has appropriate acidity in order to inhibit the proliferation of microorganisms, in addition to the purpose of enhancing taste.

Examples of the thickening agent include a suspension agent, a sedimentation agent, a gel-forming agent, a swelling agent, and the like.

The food composition of the present invention may contain a physiologically active material or minerals, which are known in the art and are stable as a food additive, for the purpose of supplementing and reinforcing functionality and nutrition, in addition to the food additive described above.

Examples of the physiologically active material include catechins contained in green tea and the like, vitamins such as vitamin B1, vitamin C, vitamin E, vitamin B12, etc., tocopherol, dibenzoyl thiamine and the like, and examples of the minerals include calcium preparations such as calcium citrate, etc., magnesium preparations such as magnesium stearate, etc., iron preparations such as iron citrate, etc. chromium chloride, potassium iodide, selenium, germanium, vanadium, zinc, and the like.

The food composition of the present invention may contain the above-mentioned food additive in an amount suitable for achieving the purpose of addition depending on the type of product.

With regard to other food additives that may be included in the food composition of the present invention, reference may be made to the Food Standards Code or the Food Additives Code of each country.

In still another specific embodiment, the antibacterial composition of the present invention may be a pharmaceutical composition to which the antibacterial active ingredient thereof is added for preservation purposes, or a pharmaceutical composition (i.e. an antibiotic composition) containing the same as an active ingredient for the prevention or treatment of a disease caused by a microorganism to be controlled. Here, examples of the disease caused by the microorganism to be controlled using the antibacterial active ingredient may include food poisoning, purulent skin, otitis media, cystitis, etc. caused by *Staphylococcus aureus;* pneumonia, etc. caused by *Klebsiella pneumoniae;* endocarditis, pneumonia, meningitis, etc. caused by *Pseudomonas aeruginosa;* and food poisoning, etc. caused by *Salmonella typhimurium.*

The pharmaceutical composition of the present invention may be manufactured in the form of an oral or parenteral formulation depending on the route of administration by typical methods known in the art, including a pharmaceutically acceptable carrier, in addition to the active ingredient. Here, the route of administration may be any suitable route including a topical route, an oral route, an intravenous route, an intramuscular route, and direct absorption through mucosal tissue, and may be used in combinations of two or more routes. An example of a combination of two or more routes is the case in which two or more drug formulations depending on the route of administration are combined, particularly, in which one drug is first administered through an intravenous route and then the other drug is administered through a topical route.

The pharmaceutically acceptable carrier is well known in the art depending on the route of administration or formulation, and specific reference may be made to the pharmacopoeia of each country, including "Korea Pharmacopoeia".

When the pharmaceutical composition of the present invention is manufactured in an oral formulation, it may be formulated into powders, granules, tablets, pills, sugar-coated tablets, capsules, liquids, gels, syrups, suspensions, wafers, and the like, in accordance with methods known in the art, together with an appropriate carrier. Here, examples of the acceptable carrier include saccharides such as lactose, glucose, sucrose, dextrose, sorbitol, mannitol, xylitol, etc., starch such as corn starch, potato starch, wheat starch, etc., celluloses such as cellulose, methyl cellulose, ethyl cellulose, sodium carboxymethyl cellulose, hydroxypropylmethyl cellulose, etc., polyvinyl pyrrolidone, water, methylhydroxybenzoate, propylhydroxybenzoate, magnesium stearate, mineral oil, malt, gelatin, talc, polyol, vegetable oil, ethanol, glycerol, and the like. When formulated, appropriate binders, lubricants, disintegrants, colorants, diluents and the like may be included as needed. Suitable binders may include starch, magnesium aluminum silicate, starch ferrite, gelatin, methylcellulose, sodium carboxymethylcellulose, polyvinyl pyrrolidone, glucose, corn sweeteners, sodium alginates, polyethylene glycols, waxes, and the like. Examples of the lubricant may include sodium oleate, sodium stearate, magnesium stearate, sodium benzoate, sodium acetate, sodium chloride, silica, talcum, stearic acid, magnesium salts and calcium salts thereof, and polyethylene glycol. Examples of the disintegrant may include starch, methyl cellulose, agar, bentonite, xanthan gum, starch, alginic acid or sodium salts thereof, and the like. Examples of the diluent may include lactose, dextrose, sucrose, mannitol, sorbitol, cellulose, glycine, etc.

When the pharmaceutical composition of the present invention is manufactured in a parenteral formulation, it may be formulated in the form of injections, transdermal delivery systems, nasal inhalers and suppositories, in accordance with methods known in the art, together with an appropriate carrier. As an appropriate carrier for an injection formulation, an aqueous isotonic solution or a suspension may be used, and specific examples thereof include isotonic solutions such as PBS (phosphate-buffered saline) containing triethanolamine, sterile water for injection, 5% dextrose, and the like. Also, when formulated in the form of a transdermal delivery system, the pharmaceutical composition of the present invention may be formulated into ointments, creams, lotions, gels, liquids for external use, pastes, liniments, aerosols, and the like. Also, for nasal inhalers, the pharmaceutical composition of the present invention may be formulated in the form of an aerosol spray using an appropriate propellant such as dichlorofluoromethane, trichlorofluoromethane, dichlorotetrafluoroethane, carbon dioxide, etc., and for suppositories, a carrier such as Witepsol, Tween 61, polyethylene glycols, cacao butter, laurin fat, polyoxyethylene sorbitan fatty acid esters, polyoxyethylene stearates, sorbitan fatty acid esters, etc. may be used.

With regard to the specific formulation of the pharmaceutical composition, reference may be made to well-known works describing the art, for example, [Remington's Pharmaceutical Sciences (19th ed., 1995)], etc., which are considered part of this specification.

The amount of the pharmaceutical composition according to the present invention, when administered, falls in the range of 0.001 mg/kg/day to 10 g/kg/day, and preferably 0.001 mg/kg/day to 1 g/kg/day, depending on the condition, weight, gender and age of the patient, the severity of the disease of the patient, and the route of administration. Administration may be carried out once a day or several times a day. The dose does not in any way limit the scope of the present invention.

In yet another specific embodiment, the composition of the present invention may be a cosmetic composition to which the active ingredient thereof is added for preservation purposes or for alleviating skin troubles caused by a microorganism to be controlled using the active ingredient thereof. Examples of the skin troubles may include purulent skin, boil, etc. of the skin caused by *Staphylococcus aureus.*

When the composition of the present invention is a cosmetic composition, the cosmetic composition may be of any product type in functional and legal classifications, and specific examples thereof may include functional cosmetics for alleviating skin troubles, non-functional general cosmetics, and the like. It may be provided in any product form, specific examples of which may include solutions, suspensions, emulsions, pastes, gels, creams, lotions, powders, soaps, surfactant-containing cleansers, oils, powder foundations, emulsion foundations, wax foundations, sprays, etc. More specific examples of the product may include skin lotions, nutritive lotions, nutritive creams, massage creams, essences, eye creams, cleansing creams, cleansing foams, cleansing water, packs, sprays, powder formulations, and the like.

The cosmetic composition of the present invention may include, in addition to the active ingredient, components typically used in cosmetic compositions, for example, typical adjuvants such as stabilizers, solubilizers, surfactants, vitamins, pigments and perfumes, and carriers.

When the formulation of the present invention is a paste, cream or gel, animal oil, vegetable oil, wax, paraffin, starch, tragacanth, a cellulose derivative, polyethylene glycol, silicone, bentonite, silica, talc or zinc oxide may be used as the carrier component.

When the formulation of the present invention is a powder or a spray, lactose, talc, silica, aluminum hydroxide, calcium silicate or a polyamide powder may be used as the carrier component. In particular, in the case of a spray, a propellant such as chlorofluorohydrocarbon, propane/butane or dimethyl ether may be additionally included.

When the formulation of the present invention is a solution or an emulsion, a solvent, solubilizer or emulsifier may be used as the carrier component. Specific examples thereof may include water, ethanol, isopropanol, ethyl carbonate, ethyl acetate, benzyl alcohol, benzyl benzoate, propylene glycol, 1,3-butylglycol oil, glycerol aliphatic ester, polyethylene glycol, fatty acid ester of sorbitan and the like.

When the formulation of the present invention is a suspension, a liquid diluent such as water, ethanol or propylene glycol, a suspension agent such as ethoxylated isostearyl alcohol, polyoxyethylene sorbitol ester or polyoxyethylene sorbitan ester, microcrystalline cellulose, aluminum metahydroxide, bentonite, agar or the like may be used as the carrier component.

When the formulation of the present invention is a surfactant-containing cleanser, aliphatic alcohol sulfate, aliphatic alcohol ether sulfate, sulfosuccinic acid monoester, isethionate, an imidazolinium derivative, methyl taurate, sarcosinate, fatty acid amide ether sulfate, alkylamido betaine, aliphatic alcohol, fatty acid glyceride, fatty acid diethanolamide, vegetable oil, a lanolin derivative or ethoxylated glycerol fatty acid ester or the like may be used as the carrier component.

The cosmetic composition of the present invention may be prepared through a method of manufacturing a cosmetic composition typically carried out in the art, with the exception that the active ingredient thereof is contained.

In still yet another specific embodiment, the composition of the present invention may be a feed composition for livestock or farmed fish, to which the active ingredient thereof is added for preservation purposes or for antibiotic purposes.

The feed composition of the present invention may be prepared so as to include, in addition to the active ingredient, typical feed components necessary for the growth of livestock or farmed fish. Examples of these components, which promote the growth of livestock or farmed fish, may include starch-containing materials, protein-containing materials, fat-containing materials, vitamin-containing materials, mineral-containing materials and the like.

The starch-containing material may generally be obtained from corn, bean, wheat, sorghum, barley, oats and the like. Suitable examples of the starch-containing material include ground/powdered corn, ground/powdered oats, ground/powdered beans, ground/powdered wheat and the like. Preferred examples of the starch-containing material include powdered/ground oats, powdered/ground corn, powdered/ground wheat, powdered/ground beans, and the like.

So long as the starch-containing material may help the growth of poultry, the amount thereof in the feed composition of the present invention is not limited to a specific range. Typically, the starch-containing material may be contained in an amount of about 30 to about 80 wt%, preferably about 40 to about 70 wt%, and most preferably 50 to 70 wt% in the feed composition of the present invention.

The protein-containing material typically includes protein-containing substances such as fish powder, bean powder, etc. Others include a bean protein concentrate, blood powder, plasma protein, skim milk dry matter, milk protein concentrate, corn gluten powder, wheat gluten powder, yeast, sunflower seed powder, etc. Preferred protein-containing materials are fish powder, blood powder, plasma protein, bean powder and the like.

So long as the protein-containing material may also help the growth of livestock or farmed fish, the amount thereof in the feed composition of the present invention is not critical. Typically, the protein-containing material may be contained in an amount of about 10 to about 50 wt%, preferably about 15 to about 40 wt%, and most preferably 18 to 30 wt% in the feed composition of the present invention.

The feed composition of the present invention may also include the fat-containing material. Suitable fat-containing materials may include, but are not limited to, lard, tallow, soybean oil, lecithin, coconut oil, and the like. Preferred fat-containing material includes soybean oil, coconut oil and lard.

So long as the fat-containing material may also help the growth of livestock or farmed fish, the amount thereof in the feed composition of the present invention is not critical. Typically, the fat-containing material may be contained in an amount of about 2 to about 20 wt%, preferably about 4 to about 15 wt%, and most preferably 6 to 12 wt% in the feed composition of the present invention.

The feed composition of the present invention may also include water-soluble and fat-soluble vitamins and minerals. Suitable vitamins include vitamin A, vitamin D, vitamin E, vitamin K, riboflavin, pantothenic acid, niacin, vitamin B12, folic acid, biotin, vitamin C and the like. Suitable minerals include copper, zinc, iodine, selenium, manganese, iron, cobalt, and the like.

When vitamins or minerals are contained in the feed composition of the present invention, the amount thereof may be set to the range of about 0.0001 to about 5 wt% based on the total weight of the feed composition of the present invention.

Yet another aspect of the present invention pertains to a poultry feed composition for egg-laying enhancement or a feed composition for weight gain, containing the fermented porcine blood described above as an active ingredient.

As is confirmed in the following examples, when feed is added with the fermented porcine blood and fed to poultry, egg-laying enhancement effects, specifically effects of increasing the egg weight and the egg-laying rate, may be exhibited, and moreover, weight gain effects may result. These effects may be directly linked to the economic benefits of poultry farms.

In the feed composition of the present invention, the fermented porcine blood may be included in the range of 0.1 wt% to 10 wt% based on the total weight of the feed composition, in consideration of the intended egg-laying enhancement and weight gain effects. The following examples show that relatively fast weight gain effects may be obtained when feed is added with 1 wt% of the fermented porcine blood and fed to poultry for 3 months. Long-term feeding of 12 months or more is expected to result in excessive weight gain and increase disease susceptibility. Accordingly, the fermented porcine blood may be added in an amount of 0.5 wt% to the feed composition for long-term feeding.

The feed composition of the present invention may be prepared so as to include, in addition to the active ingredient thereof, typical feed components necessary for the growth of poultry. Examples of these components, which promote the growth of poultry, may include starch-containing materials, protein-containing materials, fat-containing materials, vitamin-containing materials, mineral-containing materials and the like. For specific examples of these materials, reference may be made to the above description regarding the antibacterial composition of the present invention.

Still yet another aspect of the present invention pertains to a poultry egg-laying enhancement method or a poultry weight gain method, the method including feeding the fermented porcine blood described above to poultry.

In the method of the present invention, feeding the fermented porcine blood may be performed by adding the fermented porcine blood to feed or drinking water.

As described hereinbefore, the present invention is capable of providing a method of manufacturing fermented porcine blood and an antibacterial composition and a poultry feed composition using the fermented porcine blood. The antibacterial composition of the present invention can be manufactured in the form of a product, such as a quasi-drug, food, pharmaceutical, cosmetic, feed, etc. Moreover, the fermented porcine blood of the present invention can exhibit egg-laying enhancement effects and weight gain effects when fed to poultry.

### BRIEF DESCRIPTION OF DRAWINGS

FIGS. 1 and 2 show the growth characteristics of fermentation strains in the hydrolyzed blood sample and the non-hydrolyzed blood sample, respectively;
FIG. 3 shows the growth characteristics of the strain mixture depending on the amount of a carbon source that is added; and
FIG. 4 shows the growth characteristics of the strain mixture depending on the fermentation temperature.

### DESCRIPTION OF SPECIFIC EMBODIMENTS

Hereinafter, a detailed description will be given of the present invention through the following examples. However, these examples are not to be construed as limiting the scope of the present invention.

### <Examples> Preparation of fermented porcine blood and experiment on effect of fermented porcine blood as feed additive for poultry

### 1. Preparation of fermented porcine blood

Porcine blood samples were obtained from a slaughterhouse on Jeju Island, and these blood samples were added with 10% (v/v) of a 4% sodium citrate solution, serving as a blood anticoagulant, and stored frozen until the experiment.

The frozen blood sample was thawed at room temperature and added with 1% (w/v) of a proteolytic enzyme Previa™ (Novozyme, Co.), and thus hydrolyzed for 3 hr at 55°C, which is the optimal activity condition of the enzyme.

The blood sample not treated with the proteolytic enzyme or the blood sample treated with the proteolytic enzyme was added with sugar as a carbon source, mixed, inoculated with 6% (v/v) of each of five fermentation strains, namely (i) a *Lactobacillus plantarum* KCTC 21004 culture broth (2.3x10⁸ cells/ml), (ii) a *Lactobacillus agilis* KCTC 3606 culture broth (3.6x10⁸ cells/ml), (iii) a *Lactobacillus alimentarius* KCTC 3593 culture broth (2.6x10⁸ cells/ml), (iv) a *Lactobacillus fermentum* KACC 15736 culture broth (1.5x10⁸ cells/ml), and (v) a mixture of a *Lactobacillus fermentum* KACC 15736 culture broth (1.5x10⁸ cells/ml) and a *Saccharomyces cerevisiae* KCTC 7083 culture broth (4.5x10⁷ cells/ml) at 1:1, and cultured with stirring at 50 to 70 rpm, thereby manufacturing fermented porcine blood. The *Lactobacillus* culture broth was prepared in an MRS broth medium and the yeast culture broth was prepared in a YM broth medium.

### 2. Bacterial count

For bacterial count, the prepared culture broth sample was homogenized into a stock solution, and the stock solution was then diluted tenfold with a sterile saline solution (0.85% NaCl solution) to afford a working solution. 1 ml of the working solution was inoculated in a petri film medium and cultured in an incubator at 35°C for 48 hr, and the number of colonies was determined.

### 3. Strain growth and fermentation characteristics

### 3.1 Growth characteristics depending on presence or absence of proteolytic enzyme

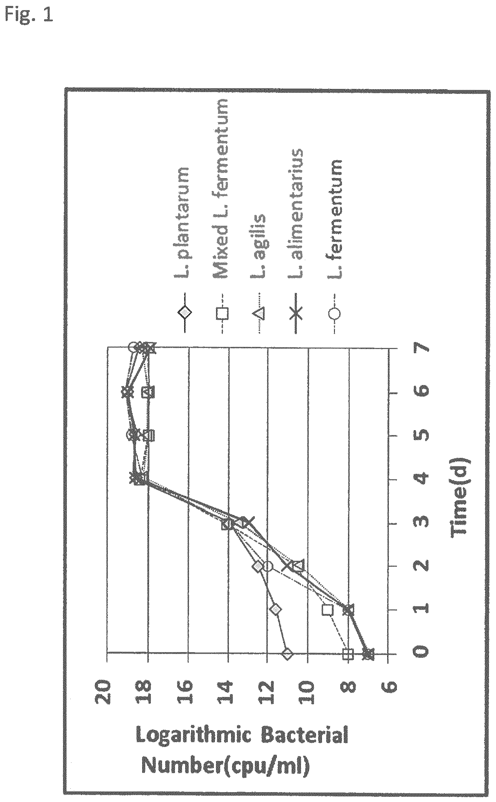

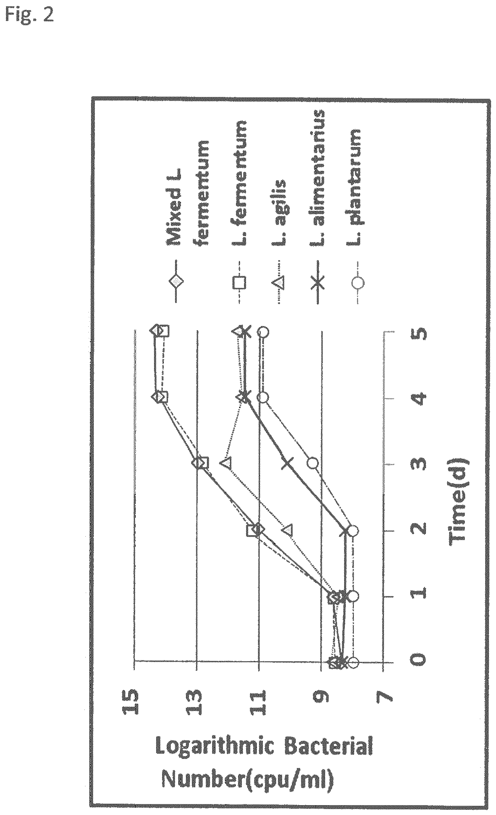

The results of culture of the hydrolyzed blood sample and the non-hydrolyzed blood sample at 30°C for 7 days using 6% (w/v) of sugar as the carbon source are shown in FIGS. 1 and 2, respectively.

As shown in FIG. 1, the hydrolyzed blood sample exhibited rapid growth of all fermentation strains and no blood coagulation.

As shown in FIG. 2, the non-hydrolyzed blood sample exhibited slow growth of all of five fermentation microorganisms, but the *Lactobacillus fermentum* strain and the strain mixture of *Lactobacillus fermentum* and *Saccharomyces cerevisiae* showed relatively fast growth. Furthermore, the culture broth began to coagulate from the 2^{nd} day after culturing and intensively coagulated on the 3^{rd} day.

In consideration of these growth characteristics, investigation of subsequent growth characteristics was performed using the strain mixture.

### 3.2 Growth characteristics depending on amount of added carbon source

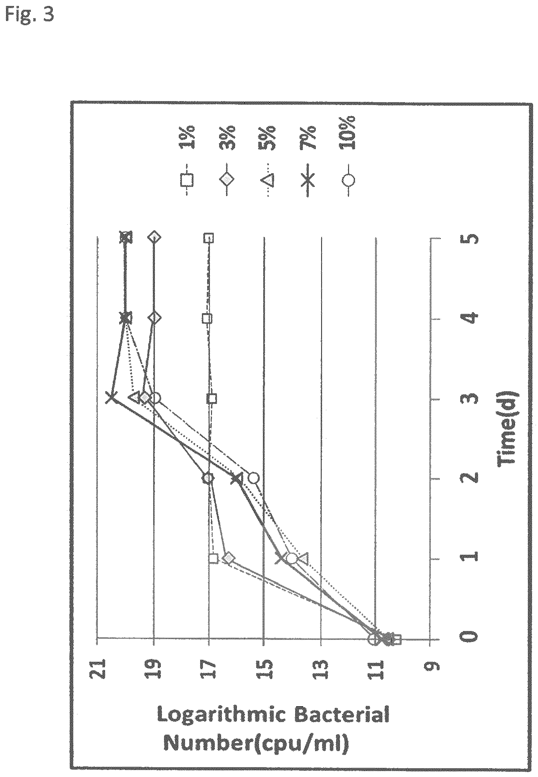

As the blood sample, the hydrolyzed blood sample was used, and as the carbon source, sugar was used in amounts of 1%, 3%, 5%, 7% and 10%, and the culturing temperature was set to 30°C. Fermentation was carried out using the strain mixture. The results of culturing for 5 days are shown in FIG. 3.

With reference to FIG. 3, in the treatment group added with 1% sugar, the growth increased until the 1^{st} day after culturing but slowed significantly from the 2^{nd} day. In the treatment group added with 3% sugar, rapid growth appeared until the 3^{rd} day, after which growth also slowed significantly. The groups added with 5% or more of sugar manifested continuous growth after culturing and rapid growth up to the 4^{th} day. Therefore, for economic fermentation, the concentration of sugar that was added was determined to be in the range of 5 to 7%.

### 3.3 Growth characteristics depending on fermentation temperature

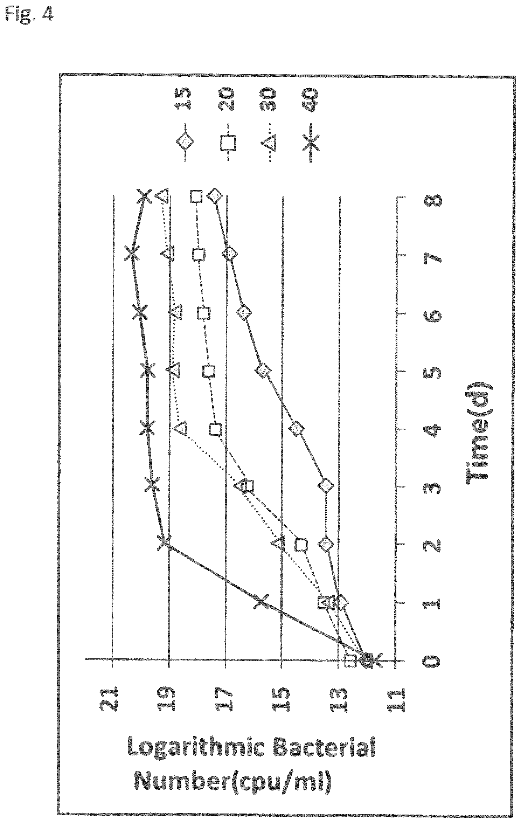

As the blood sample, the hydrolyzed blood sample was used, the carbon source was 6% sugar, and the fermentation temperature was set to 15°C, 20°C, 30°C and 40°C. Fermentation was carried out using the strain mixture. The results of culturing for 8 days are shown in FIG. 4.

With reference to FIG. 4, the treatment group at 15°C showed gentle growth during the culturing period and the lowest growth characteristics at the end of culturing. The treatment group at 20°C manifested gentle growth and then the growth slowed and appeared to be stable on the 4^{th} day. In the treatment group at 30°C, relatively fast growth was observed, and on the 4^{th} day, the stable stage appeared, and the bacterial count reached a level of 10⁸ cells/ml. The treatment group at 40°C showed rapid growth until the 2^{nd} day, reaching a level of 10⁸ cells/ml, and from the 3^{rd} day it reached the stable stage. Therefore, growth characteristics tended to increase with an elevation in the temperature. The appropriate fermentation temperature was determined to be 30°C or higher taking into consideration economic benefits and fermentation rate.

### 3.4 Fermentation characteristics under optimal fermentation conditions

The characteristics of the fermented porcine blood obtained under the optimal fermentation conditions confirmed above, particularly use of the hydrolyzed blood sample, use of the strain mixture, use of 6% carbon source sugar, a fermentation temperature of 30°C and fermentation for a total of 6 days, are summarized in Table 1 below.

As is apparent from Table 1 below, on the 3^{rd} day after the start of fermentation (October 27), the fermentation was judged to reach an end point based on the number of bacteria, pH, fermentation odor and color. From the 4^{th} day to the 6^{th} day, the number of bacteria increased slightly, and the pH was kept constant at 4.0, which appeared to indicate a stable stage, rather than a growth stage. In particular, long-term storage of about 12 months at room temperature was determined to be possible because of the constant pH of 4.0.

**[Table 1]**

| Culture date | October 24 | October 25 | October 26 | October 27 | October 28 | October 29 | October 30 |
|---|---|---|---|---|---|---|---|
| Time (d) | 0 | 1 | 2 | 3 | 4 | 5 | 6 |
| pH | 7.5 | 6.9 | 4.7 | 4.2 | 4 | 4 | 4 |
| Viscosity | 85 | 80 | 77 | 79 | 70 | 78 | 72 |
| Color | Red | Red | Blackisn brown | Dark blackish brown | Dark blackish brown | Dark blackish brown | Dark blackish brown |
| Odor | Blood odor | Blood odor | Fermentation odor | Fermentation odor | Fermentation odor | Fermentation odor | Fermentation odor |
| Number of microorganisms (cpu/ml) | 3.5x10⁵ | 2.1x10⁶ | 7.3x10⁷ | 2.9x10⁸ | 3.8x10⁶ | 3.0x10⁸ | 3.6x10⁸ |

Also, the results of general analysis are shown in Table 2 below. As is apparent from Table 2, heavy metals such as lead, mercury and the like were not detected, and no antibiotics were detected. These results were found to satisfy the Standards and Specifications of Feed and the like under the Ministry of Agriculture, Food and Rural Affairs.

**[Table 2]**

| Analysis items | Unit | Fermentation stock solution |
|---|---|---|
| Water | % | 80.11 |
| Crude protein | % | 14.97 |
| Crude fat | % | 0.43 |
| Crush ash | % | 0.75 |
| Calories | cal/g | 1157 |
| Calcium (Ca) | mg/kg | 602.21 |
| Chromium (Cr) | mg/kg | Not detected |
| Copper (Cu) | mg/kg | Not detected |
| Iron (Fe) | mg/kg | 176.51 |
| Potassium (K) | mg/kg | 1055.87 |
| Magnesium (Mg) | mg/kg | 123.54 |
| Manganese (Mn) | mg/kg | Not detected |
| Sodium (Na) | mg/kg | 488.83 |
| Phosphorus (P) | mg/kg | 403.56 |
| Zinc (Zn) | mg/kg | 5.46 |
| Salt | % | 0.32 |
| Cadmium (Cd) | mg/kg | 0.07 |
| Lead (Pb) | mg/kg | Not detected |
| Mercury (Hg) | mg/kg | Not detected |
| Arsenic (As) | mg/kg | 1.68 |
| Sulfur (S) | % | 0.12 |
| Acetic acid | % | 0.45 |
| Butyric acid | % | 0.13 |
| L-lactic acid | % | 1.76 |
| Propionic acid | % | Not detected |
| Beta-lactam | | Negative |
| Macrolide | | Negative |
| Sulfonamide | | Negative |
| Aminoglycoside | | Negative. |
| Tetracycline | | Negative |

### 4. Antibacterial experiment

### 4.1 Antibacterial experiment method

An antibacterial experiment was performed in accordance with ASTM E149, and is as follows.

1 ml of the sample was mixed with a phosphate buffer (pH 7.0) and added with the test bacterial culture broth so as to attain a concentration of 2.5x10⁵ cfu/ml. The total volume of the phosphate buffer to which the sample and the test bacteria were added was adjusted to 50 ml so that the concentration of the sample that was added was 2% (v/v), and 1 ml of the diluted solution was spread on a film medium for bacterial culture and then cultured for 24 hr. Finally, the number of test bacteria in the cultured film medium was measured.

### 4.2 Antibacterial experiment results

The antibacterial experiment results are shown in Table 3 below.

**[Table 3]**

| Antibacterial experiment results | | | | |
|---|---|---|---|---|
| Classification | | Blank | 40Bx sample | 50Bx sample |
| *E. coli* | Initial bacterial count | 2.1x10⁵ | 2.1x10⁵ | 2.1x10⁵ |
| | After 24 hr | 1.3x10⁵ | < 30 | < 30 |
| | Bacterial reduction rate | - | 99.9 | 99.9 |
| *S. aureus* | Initial bacterial count | 2.1x10⁵ | 2.1x10⁵ | 2.1x10⁵ |
| | After 24 hr | 1.3x10⁵ | 2.8x10² | 3.5x10⁴ |
| | Bacterial reduction rate | - | 99.8 | 73.1 |
| *K. pneumoniae* | Initial bacterial count | 2.1x10⁵ | 2.1x10⁵ | 2.1x10⁵ |
| | After 24 hr | 1.7x10⁵ | < 30 | < 30 |
| | Bacterial reduction rate | - | 99.9 | 99.9 |
| *P. aeruginosa* | Initial bacterial count | 2.1x10⁵ | 2.1x10⁵ | 2.1x10⁵ |
| | After 24 hr | 1.3x10⁵ | < 30 | < 30 |
| | Bacterial reduction rate | - | 99.9 | 99.9 |
| *S. typhimurium* | Initial bacterial count | 2.1x10⁵ | 2.1x10⁵ | 2.1x10⁵ |
| | After 24 hr | 1.7x10⁵ | < 30 | < 30 |
| | Bacterial reduction rate | - | 99.9 | 99.9 |
| * Sample concentration: 2% (v/v) | | | | |
| Bacterial reduction rate unit: % | | | | |
| Bacterial count unit: cells/ml | | | | |
| Buffer solution: 50 ml of Phosphate buffer (pH 7.2) | | | | |
| Control sample: non-fermented blood (sample vacuum-concentrated to 40 Brix) | | | | |

The results of Table 3 show that the fermented blood sample exhibit very high antibacterial activity against all bacteria tested. For reference, the non-fermented blood sample (obtained through treatment with sodium citrate and then hydrolysis with proteolytic enzyme) showed no antibacterial activity at all, but all of the above bacteria proliferated, and moreover, severe decay was visible with the naked eye (data not shown).

### 5. Poultry farm site evaluation

### 5.1 Experiment method

The weight evaluation of broilers (variety: Hanhyup 3) was performed from November 1, 2018 to March 2, 2019.

The evaluation of egg-laying rate of laying hens (variety: Hy-Line Brown, type: brown species, brown eggs) was carried out from February 1, 2019 to March 2, 2019.

As feed, feed for laying hens and broilers from Dongyang Feed (Gyeonggi, Korea) (control group) and a mixture thereof with 1% (w/w) of the lyophilized powder of fermented porcine blood obtained under optimal fermentation conditions (treatment group) were used.

The feed was freely provided during the experiment, and drinking water was freely provided using an automatic water dispenser.

### 5.2 Experiment results

The results of weight evaluation of broilers are shown in Table 4 below, and the results of evaluation of egg-laying rate and the like of laying hens are shown in Table 5 below.

As is apparent from Table 4, the weight gain rate during the experiment was 6.9% in the control group but was apparently increased to 13.3% in the treatment group.

As is apparent from Table 5, the number of eggs and the egg-laying rate during the experiment were significantly increased.

**[Table 4]**

| Classification | Weight at start date of experiment (g) | Weight at end date of experiment (g) | Weight gain (g) | Weight gain rate |
|---|---|---|---|---|
| Control group (20 broilers) | 40,150 | 42,900 | 2,750 | 6.8% |
| Treatment group (20 broilers) | 40,300 | 45,630 | 5,330 | 13.2% |

**[Table 5]**

| Classification | Mortality | Breeding (hens) | Eggs (number) | Egg weight (40, g) | Total feed intake (4C, g) | Daily feed intake per hen (g) | Egg-laying rate (%) | Feed efficiency | Feed requirement |
|---|---|---|---|---|---|---|---|---|---|
| Control group | 1 | 39 | 37^{a} | 2,278* | 4,613 | 115.33^{a} | 94.43⁺ | 0.494 | 2.029 |
| Treatment group | 0 | 40 | 39^{a,b} | 2,416** | 4,919 | 122.97^{a,b} | 96.83⁺⁺ | 0.491 | 2.038 |

| | | | | | | | | | |
|---|---|---|---|---|---|---|---|---|---|
| a, b, ab: T-Test, P<0.05 *, **: T-Test, P<0.05 +, ++: T-Test, P<0.05 | | | | | | | | | |

Although preferred embodiments of the present invention have been disclosed for illustrative purposes, those skilled in the art will appreciate that various modifications are possible without departing from the scope and spirit of the invention as disclosed in the accompanying claims, and such modifications should not be understood separately from the technical ideas or essential characteristics of the present invention.

## Claims

1. A method of manufacturing fermented porcine blood, the method comprising:
(a) preparing porcine blood; and
(b) subjecting the porcine blood to inoculation with a fermentation strain selected from among *Lactobacillus plantarum, Lactobacillus agilis, Lactobacillus alimentarius, Lactobacillus fermentum* and a strain mixture of *Lactobacillus fermentum* and *Saccharomyces cerevisiae,* and to fermentation culture.

2. The method of claim 1, wherein the porcine blood is added with a blood anticoagulant, and
hydrolyzing the porcine blood with a proteolytic enzyme is performed after step (a) and before step (b), whereby the inoculation with the fermentation strain is carried out for the porcine blood hydrolyzed with the proteolytic enzyme.

3. The method of claim 2, wherein the hydrolyzed porcine blood is added with 5 to 7 wt% of a carbon source, and the culture is carried out at 30°C to 45°C.

4. A fermented porcine blood, obtained by the method of any one of claims 1 to 3.

5. An antibacterial composition, comprising the fermented porcine blood of claim 4 as an active ingredient.

6. The composition of claim 5, which has antibacterial activity against *E. coli, Staphylococcus aureus, Klebsiella pneumoniae, Pseudomonas aeruginosa* and *Salmonella typhimurium.*

7. The composition of claim 5, wherein the composition is a quasi-drug composition.

8. The composition of claim 5, wherein the composition is a pharmaceutical composition.

9. The composition of claim 5, wherein the composition is a cosmetic composition.

10. The composition of claim 5, wherein the composition is a food composition.

11. The composition of claim 5, wherein the composition is a feed composition.

12. A poultry feed composition for egg-laying enhancement, comprising the fermented porcine blood of claim 4 as an active ingredient.

13. A poultry feed composition for weight gain, comprising the fermented porcine blood of claim 4 as an active ingredient.

14. A poultry egg-laying enhancement method, the method comprising feeding the poultry feed composition of claim 12 to poultry.

15. A poultry weight gain method, the method comprising feeding the poultry feed composition of claim 13 to poultry.
